# EUROPEAN PATENT APPLICATION

(11) **EP 1 262 209 A1**
(43) Date of publication of application: **04.12.2002**
(21) Application number: 02253535.5
(22) Date of filing: 20.05.2002
(51) Int. Cl.: A61M 16/16

(54) **Device for increasing the humidification of the air stream breathed by a patient**

(30) Priority: 22.05.2001 IT MI20011073
(71) Applicant: Mallinckrodt Holdings B.V., 7400 AA Deventer (NL)
(72) Inventor: Fini, Massimo, 41037 Mirandola (IT); Mambrini, Giovanni, 41033 Concordia (IT)
(74) Representative: Jones, Alan John

(57) **Abstract**

A device for increasing the humidification of the air stream breathed by a patient, having a tubular body (2) that can be coupled to a heat and moisture exchange filter (3) and forms a channel (10) for the passage of an air stream. At least one portion of the channel (10) is provided by a membrane (11) impermeable to gases and permeable to ions and molecules with a partial charge. The membrane (11) delimits an interspace (15) that is external to the channel (10) and allows passage of a water vapor flow.

## Description

The present invention relates to a device for increasing the humidification of the air stream breathed by a patient.

It is known that the initial treatment of the air or gases inspired by a person normally occurs in the upper respiratory tract, i.e., in the so-called upper air tracts or ways; accordingly, in the case of patients subjected to mechanical ventilation with an endotracheal tube the contribution of the upper air tracts or ways is excluded completely and it is therefore necessary to humidify the air breathed by the patient.

This phenomenon is further exacerbated by the use of medical air, which due to technical requirements is particularly filtered and dry, with an absolute humidity of approximately 1 mg/l, thereby it is absolutely necessary to humidify the air stream in order to avoid the risk of causing lesions to the pulmonary epithelium of the patient, which would gradually worsen over time, becoming irreversible after a few hours.

In order to solve this problem, filtration devices for passive humidification, termed HME (Heat and Moisture Exchanger), are already known; they retain a significant fraction of the moisture and heat expired by the patient in order to return the moisture during inspiration.

This phenomenon has an efficiency of approximately 80% and offers no additional contribution in terms of humidity or heat with respect to what the patient exhales.

Devices are already known which allow to increase the humidification of the air, and for this purpose appropriate humidifiers are usually provided which in practice drip water onto the filtration element, which must be heated appropriately so as to facilitate the evaporation of the added water.

This approach causes considerable complications, since it requires connecting both a heating element and the dripping element to the filter.

The aim of the present invention is to eliminate the drawbacks mentioned above, by providing a device for increasing the humidification of the air stream breathed by a patient which also allows to provide an addition of heat without having to provide heat sources located proximate to the HME filter.

Within this aim, an object of the invention is to provide a device that is particularly lightweight and therefore does not increase the weight of the devices to be connected to the patient.

Another object of the present invention is to provide a device which, by way of its particular constructive characteristics, is capable of giving the greatest assurances of reliability and safety in use.

Another object of the present invention is to provide a device that can be obtained easily starting from commonly commercially available elements and materials and is further competitive from a merely economical standpoint.

This aim and these and other objects that will become better apparent hereinafter are achieved by a device for increasing the humidification of the air stream breathed by a patient, according to the invention, characterized in that it comprises a tubular body that can be coupled to a heat and moisture exchange filter and forms a channel for the passage of an air stream, at least one portion of the channel being provided by way of a membrane that is impermeable to gases and permeable to ions and molecules with a partial charge, the membrane delimiting an interspace that is external to the channel and allows passage of a water vapor flow.

Further characteristics and advantages of the present invention will become better apparent from the following detailed description of a preferred but not exclusive embodiment of a device for increasing the humidification of the air stream breathed by a patient, illustrated only by way of non-limitative example in the accompanying drawings, wherein:
Figure 1 is a schematic sectional view of the device according to the invention;
Figure 2 is a view of the ventilation unit to which the device according to the invention is applied.

With reference to the figures, the device for increasing the humidification of the air stream breathed by a patient, according to the invention, generally designated by the reference numeral 1, comprises a tubular body 2, which is interposed between the heat and moisture exchange filter 3, of a per se known type, and the endotracheal tube 4 that is connected to the patient.

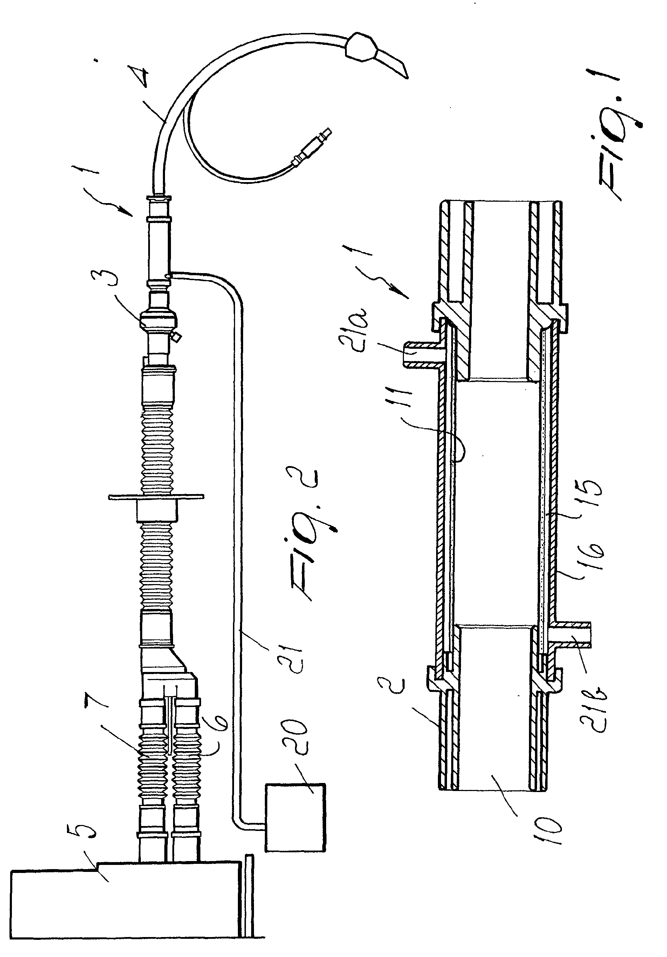

The filter 3, as shown in Figure 2, is arranged in a per se conventional manner downstream of the ventilation unit 5 from which the expiratory duct 6 and inspiratory duct 7 branch out.

The particularity of the invention consists in that the tubular body 2 defines or forms internally a channel 10 for the passage of the respiratory air stream; at least one portion of the channel 10 is provided by a membrane 11 that is impermeable to gases and permeable to ions and molecules with a partial charge.

Such membrane is provided by a tubular portion made of a copolymer of perfluorosulfonic acid and polytetrafluoroethylene in acid form; for example, a material of this kind is known commercially under the trademark Nafion by DuPont.

The membrane 11 delimits an interspace 15, in cooperation with an outer enclosure 16 provided on the tubular body 2, through which saturated-type water vapor flows; the membrane 11 allows the water to pass in the form of a molecule that is locked in a permanent dipole state and therefore has ionic characteristics in the molecular state, but does not allow the passage of all the other gases involved in respiration; accordingly, the Nafion membrane allows water to pass from a high-concentration environment to a lower-concentration environment until the intended equilibrium is reestablished.

The tubular portion made of Nafion can therefore increase the humidification of the air stream inside the channel 10 and also allows to heat the gases of the air stream, which accordingly reach the patient not only after being enriched with moisture but also after being duly heated.

In order to produce the stream of water vapor, there is a humidifier-heater 20, in which a heater acts as a source of vapor and a small centrifugal pump allows constant recycling of the warm saturated vapor by means of a double-lumen tube 21 that is connected to the inlet 21a and the outlet 21b, which are connected to the interspace 15.

With the device described above it is therefore possible to provide continuous additional humidification of the air by using water vapor as a replacement of known devices, which required the use of a heating system inside the respiration duct and dripping on the filter.

From the above description it is evident that the invention achieves the intended aim and objects, and in particular the fact is stressed that a device for increasing the humidification of the air stream breathed by a patient is provided which is particularly compact and easy to handle and allows, by way of the use of the semipermeable membrane made of Nafion, to achieve a passage of water while blocking the flow of gases through it; it should also be noted that the Nafion membrane is impermeable to viruses and bacteria.

The invention thus conceived is susceptible of numerous modifications and variations, all of which are within the scope of the appended claims.

All the details may further be replaced with other technically equivalent elements.

In practice, the materials used, as well as the contingent shapes and dimensions, may be any according to requirements.

The disclosures in Italian Patent Application No. MI2001A001073 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for increasing the humidification of the air stream breathed by a patient, **characterized in that** it comprises a tubular body (2) that can be coupled to a heat and moisture exchange filter (3) and forms a channel (10) for the passage of an air stream, at least one portion of said channel (10) being provided by way of a membrane (11) that is impermeable to gases and permeable to ions and molecules with a partial charge, said membrane (11) delimiting an interspace (15) that is external to said channel (10) and allows passage of a water vapor flow.

2. The device according to claim 1, **characterized in that** said tubular body (2) is interposed between said heat and moisture exchange filter (3) and the patient.

3. The device according to the preceding claims, **characterized in that** said membrane (11) is provided as a tubular portion that is made of a copolymer of perfluorosulfonic acid and polytetrafluoroethylene in acid form.

4. The device according to one or more of the preceding claims, **characterized in that** said membrane (11) is made of a commercially available type of copolymer of perfluorosulfonic acid and polytetrafluoroethylene in acid form.

5. The device according to one or more of the preceding claims, **characterized in that** said interspace (15) is delimited by said membrane (11) and by an outer enclosure (16) formed on said tubular body (2).

6. The device according to one or more of the preceding claims, **characterized in that** said interspace (15) is connected to a humidifier-heater (20) for producing and introducing saturated steam suitable to act both as a humidification element and as a heating element.
